# EUROPEAN PATENT SPECIFICATION

(11) **EP 3 814 364 B1**
(45) Date of publication and mention of the grant of the patent: **12.08.2026**
(21) Application number: 19820440.6
(22) Date of filing: 07.01.2019
(51) Int. Cl.: C07H 15/18

(54) **IMPROVED PROCESS FOR PREPARATION OF 2,3,4,6-TETRA-O-BENZYL-D-GALACTOSE**
VERBESSERTES VERFAHREN ZUR HERSTELLUNG VON 2,3,4,6-TETRA-O-BENZYL-D-GALACTOSE
PROCÉDÉ AMÉLIORÉ DE PRÉPARATION DE 2,3,4,6-TÉTRA-O-BENZYL-D-GALACTOSE

(30) Priority: 11.06.2018 IN 201821021783
(43) Date of publication of application: 05.05.2021
(73) Proprietor: Aarti Pharmalabs Limited, Gujarat 396195 (IN)
(72) Inventor: DESAI, Parimal Hasmukhlal, Mumbai 400080, Maharashtra (IN); PATRAVALE, Bharatkumar Surendra, Mumbai- 421 204, Maharashtra (IN); KULKARNI, Guruprasad Manohar, Mumbai- 421 204, Maharashtra (IN); KAJALE, Nitin Baburao, Mumbai- 421 204, Maharashtra (IN); CHAUDHARI, Subodh Vasant, Mumbai- 421 204, Maharashtra (IN)
(74) Representative: Crease, Devanand John
(86) International application number: PCT/IN2019/050010
(87) International publication number: WO 2019/239425

(56) References cited:
- CN-A- 103 665 064
- CN-B- 103 694 288
- JP-A- 2006 083 091
- GOLA, G.; LIBENSON, P.; GANDOLFI-DONADÍO, L.; GALLO-RODRIGUEZ, C.: "Synthesis of 2,3,5,6-tetra-O-benzyl-D-galactofuranose for -glycosidation", ARKIVOC, vol. 2005, no. 12, 2005, pages 234 - 242, XP055759089, ISSN: 1424-6376

## Description

### Field of the Invention

The present invention relates to generally to a process for preparation of benzylated derivative of D-galactose, and more particularly to an improved process for preparation of 2,3,4,6-tetra-O-benzyl-D-galactose with higher yield and better purity with reduced impurities.

### Background of the Invention

Methyl-D-galactopyranoside and benzylated derivative of D-galactose are used as precursor for synthesis of many active pharmaceutical ingredient and intermediates thereof. The compounds are used in the synthesis of Cholestan, deoxygalactonojirimycin such as Lucerastat and the like. Pure form of 2,3,4,6-Tetra-O-benzyl-D-galactose (A) is very important, as its end use is as precursor for synthesis of many active pharmaceutical ingredient and intermediates.

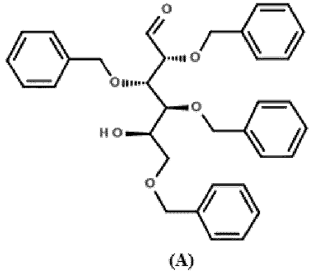

Carbohydrate Research, 345(12), 1663-1684; 2010 discloses preparation of 2,3,4,6-tetra-O-benzyl-D-galactopyranoside by reacting Methyl-D-Galactopyranoside with benzyl bromide in presence of sodium hydride as base and in dimethyl formamide as solvent. However the yield obtained is very low in the range of 25-50%.

JP2006083091 discloses benzylation of Methyl-D-galactopyranoside using 6 equivalents of benzyl bromide in presence of 6 equivalents of sodium hydride in dimethyl formamide to form 2,3,4,6-tetra-O-benzyl-D-galactopyranoside. The yield reported is 70%, however the reaction time is 17 hours and also work up part is very lengthy, which is not possible at higher scale production.

Sodium hydride is commercially available as dispersion in paraffin oil, hence tetrabenzyl monosaccaride obtained is with paraffin oil as impurity. Sodium hydride is inflammable and also has the risk of storage, transport and use of the process, especially at large scale production. In reaction with sodium hydride, exotherm reaction is observed during addition of benzyl halide, during work up after addition of water and also during quenching.

Journal of Carbohydrate Chemistry, 6(4), 645-660, 1987 discloses synthesis of perbenzylated methyl-4-D-mannopyranoside by using inexpensive commercially available methyl α-d-mannopyranoside as starting material. methyl α-d-mannopyranoside (1 mmol) was treated with Benzyl chloride (7.3 mmol) and powdered KOH (11.6 mmol). The reaction is carried out in 4-5 volume of solvent DMSO at room temperature. The completion of reaction required minimum 15 hours and yields reported is 66-87%. The time required for the completion of reaction is 15 hours with the given number of moles of benzyl bromide and alkali. Although the yield reported is around 66-87%, it is contaminated with mono-, di-, tri-, penta-benzylated Galactose impurities.

CN 103 694 288 and CN 103 665 064 both describe further proceeses to prepare high purity 2,3,4,6-tetra-O-benzyl galactose.

It is difficult to achieve purity of 2,3,4,6-Tetra-O-benzyl-D-galactose with general purification techniques. As the product is contaminated with mono-, di-, tri-, penta-benzylated galalctose impurities and dibenzyl ether impurity, hence removal of these impurities requires repeated purifications which ultimately results in product loss. Also due to similar properties as of product, the separation of impurities from the product is very difficult. Thus a specific purification technique is needed for without affecting yield of the product.

Thus there is need to develop a process for O-benzylation of galactose considering factors such as perfect stoichiometry of the base as per nature of the base, technique and time of addition of reagents and optimization of reaction conditions for effective O-benzylation. Further, there is a need for an improved process for preparation of methyl-D-galactopyranoside and benzylated derivative of D-galactose which overcomes the above mentioned drawbacks of the prior art.

### Objects of the invention

An object of the present invention is to provide an improved process for preparation of benzylated derivative of D-galactose, particularly 2,3,4,6-tetra-O-benzyl-D-galactose that gives higher yield and better purity.

Another object of the present invention is to provide the improved process for preparation of benzylated derivative of D-galactose, particularly 2,3,4,6-Tetra-O-benzyl-D-galactose which involves use of safer reagents that are easy to store and industrialize.

### Summary of the invention

In a general aspect, the present invention relates to an improved process for the preparation of a pure form of a compound of formula (A).

Also disclosed herein is a purification process of a compound of formula (IV) free from impurities and to achieve high quality pure form of a compound of formula (A).

According to the invention, a process for preparation of a compound of formula (A) is disclosed.

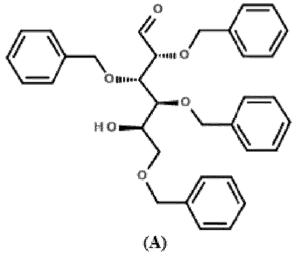

The process comprises:
a. O-methylation of a compound of formula (I) to a compound of formula (II) 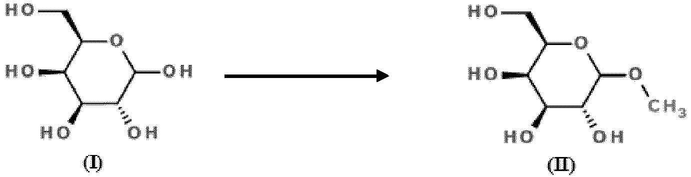 in presence of a predefined catalyst and a predefined solvent at a predefined heating temperature;
b. reacting the compound of formula (II) in a predefined polar aprotic solvent in presence of a predefined phase transfer catalyst to obtain a reaction mixture; and adding a predefined base and a predefined reagent to the reaction mixture at a predefined temperature to form a compound of formula (III); 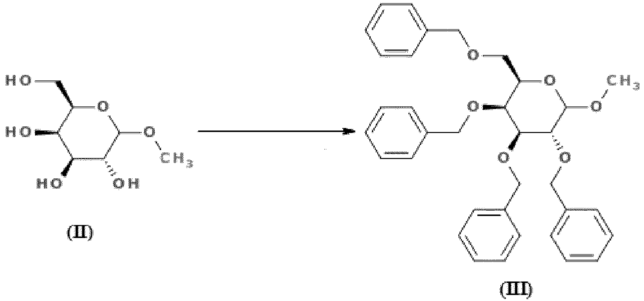
c. converting the compound of formula (III) to a compound of formula (IV) 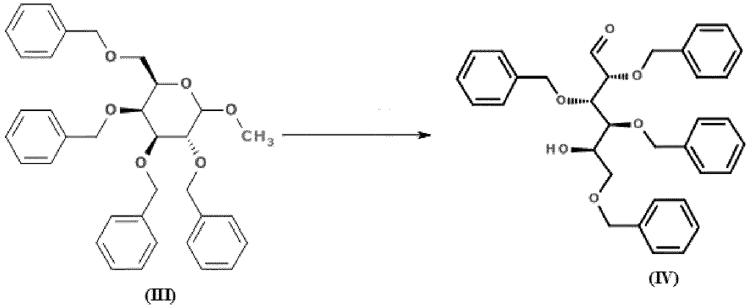 in presence of a mixture of a predefined acid at a predefined temperature;
d. purifying the compound of formula (IV) to a compound of formula (A) 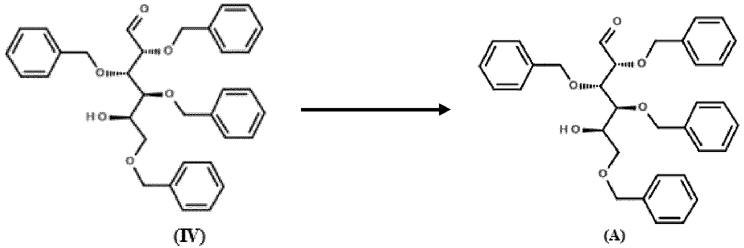 said purification process comprising the steps of:
   i. acylating the compound of formula (IV) to form a compound of formula (V) 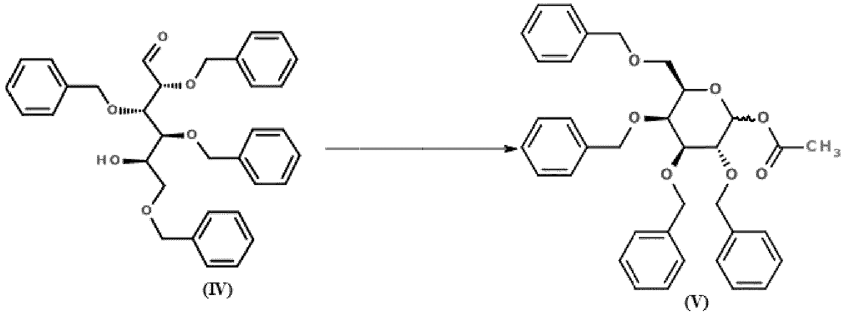 using a predefined acylating agent in presence of a predefined base and a predefined solvent at a predefined temperature; and
   ii. hydrolysis of the compound of formula (V) to form the compound of formula (A) 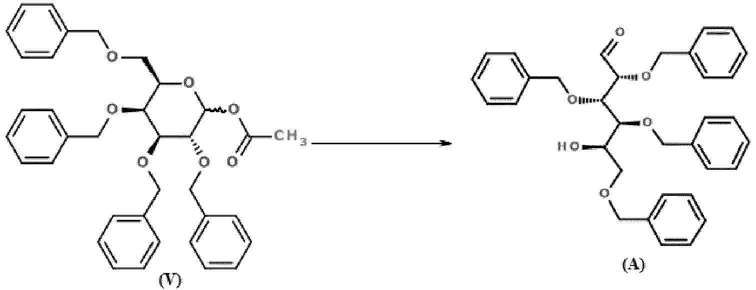 in presence of a predefined base and a predefined solvent at a predefined temperature.

In an embodiment, the predefined catalyst in step (a.) is selected from the group of hydrochloric acid and sulfuric acid. The heating temperature may be from 65°C to 70°C. In an embodiment, the predefined polar aprotic solvent in step (b.) is selected from the group of dimethyl formamide (DMF), dimethyl sulfoxide (DMSO), tetrahydrofuran, preferably dimethyl sulfoxide (DMSO). In an embodiment, the predefined phase transfer catalyst in step (b.) is selected from the group of tetra-n-butylammonium chloride (TBAC), tetra-n-butylammonium bromide (TBAB), triethylbenzylammonium chloride (TEBA), methyltrioctylammonium chloride (Aliquat-336), cetyltriethylammonium chloride (CTEAL), benzyltributylammonium chloride (BTBAC1), tetra-n-butylammonium fluoride (TBAF), preferably tetra-n-butylammonium bromide (TBAB). In an embodiment, the predefined base in step (b.) is selected from the group of potassium hydroxide, sodium hydroxide, sodium methoxide, sodium carbonate and potassium tert-butoxide, preferably potassium hydroxide. In an embodiment, the predefined reagent in step (b.) is selected from the group of benzyl halide wherein the benzyl halide is selected from the group of benzyl chloride, benzyl bromide and benzyl iodide. In an embodiment, the amount of the base in step (b.) used is in the range of 4.5 molar equivalent to 24 molar equivalent, preferably from 6 molar equivalent to 16 molar equivalent, and more preferably 9 molar equivalent. The amount of the reagent in step (b.) may be in the range of 5 molar equivalent to 15.5 molar equivalent, preferably 5 molar equivalent to 10 molar equivalent, and more preferably 5.7 molar equivalent. The predefined temperature of the reaction in step (b.) may range from 5°C to 30°C, preferably from 10°C to 25°C, and more preferably from 15°C to 20°C. In an embodiment, the addition of the predefined reagent with the predefined base in step (b.) is done lot-wise over a predefined period of time from 40 minutes to 45 minutes and at a predefined temperature ranging from 15°C to 20°C in each lot. In an embodiment, the predefined acid in step (c.) is selected from the group of hydrobromic acid, hydrochloric acid, sulfuric acid and acetic acid. In an embodiment, the predefined mixture of the predefined acid is selected from sulfuric acid and acetic acid. The predefined ratio of the mixture of the predefined acid may be 0.96:6 to 9.6:17. The predefined volume/volume percent of acetic acid used may range from 6 v/v % to 17 v/v %, preferably from 6 v/v % to 10 v/v %, and more preferably 6.75 v/v %. The predefined quantity of sulfuric acid used may range from 0.1 to 1 molar equivalent, and preferably 0.16 molar equivalent. The temperature of reaction in step (c.) may be carried out in the range of 90°C to 110°C, and preferably at a range of 100°C to 105°C. In an embodiment, the predefined acylating agent in step (d.) (i.) is selected from the group of acetic acid and acetic anhydride. In an embodiment, the predefined base in step (d.) (i.) is selected from the group of triethylamine and di-isopropylethylamine (DIPEA). In an embodiment, the predefined solvent in step (d.) (i.) is selected from the group of chlorinated solvents selected from the group of chloroform, dichloromethane, dichloroethane, preferably dichloromethane. In an embodiment, the reaction temperature range in step (i.) is from 25°C to 30°C. In an embodiment, the predefined base in step (d.) (ii.) is selected from the group of predefined carbonates selected from the group of sodium carbonate, potassium carbonate, calcium carbonate, aluminium carbonate, ammonium carbonate, caesium carbonate, barium carbonate, magnesium carbonate, lithium carbonate and bicarbonates selected from the group of sodium bicarbonate, potassium bicarbonate, caesium bicarbonate, calcium bicarbonate, ammonium bicarbonate, magnesium bicarbonate. In an embodiment, the predefined solvent in step (d.) (ii.) is selected from the group of solvent methanol, ethanol, propanol, isopropanol, n-butanol, iso-butanol, toluene, o-xylene, m-xylene, p-xylene, ethylbenzene, 4-ethyl toluene, dimethyl benzene, trimethyl benzene, cymene, and/or mixtures thereof. In an embodiment, in step (d.) (ii.), a predefined mixture of methanol and toluene is used. The predefined volume/volume percent of methanol ranges from 4 v/v % to 8 v/v % and toluene ranges from 4 v/v % to 8 v/v % and the predefined volume of methanol is preferably 5 v/v % and the predefined volume/volume of toluene is preferably 5 v/v % wherein the ratio of methanol to toluene may be 0.5:1 to 1:0.5. The compound of formula (A) has HPLC purity of 97% to 99.5% with yield of 50% to 80%.

### Detailed description of the invention

The foregoing objects of the present invention are accomplished and the problems and shortcomings associated with the prior art, techniques and approaches are overcome by the present invention as described below in the preferred embodiments.

All materials used herein were commercially purchased as described herein or prepared from commercially purchased materials as described herein.

Although specific terms are used in the following description for sake of clarity, these terms are intended to refer only to particular structure of the invention selected for illustration in the drawings and are not intended to define or limit the scope of the invention.

References in the specification to "preferred embodiment" means that a particular feature, structure, characteristic, or function described in detail thereby omitting known constructions and functions for clear description of the present invention.

The stoichiometry of base required for O-benzylation of carbohydrates is not generalized and is specific to the carbohydrate substrate. The stoichiometry and the nature of base claimed and the conditions under which O-alkylation is carried out with high yields is specific to galactose and relates to its geometry and stability under the reaction conditions adopted. The reaction conditions for effective O-alkylation of galactose is specific to it and procedures for similar conversion reported for other carbohydrate substrates are not applicable in its entirety.

In an embodiment of the present invention, an improved process for a preparation of a pure form of a compound of formula (A) is disclosed.

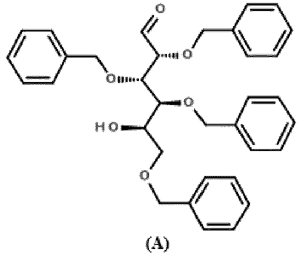

The process comprising:
a. O-methylation of a compound of formula (I) to a compound of formula (II) 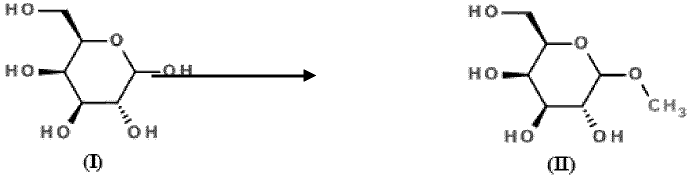 in presence of a predefined catalyst and a predefined solvent at a predefined heating temperature;
b. reacting the compound of formula (II) in a predefined polar aprotic solvent in presence of a predefined phase transfer catalyst to obtain a reaction mixture; and adding a predefined base and a predefined reagent to the reaction mixture at a predefined temperature to form a compound of formula (III); 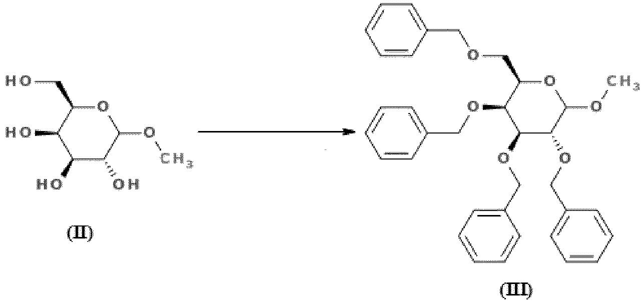
c. converting the compound of formula (III) to a compound of formula (IV) 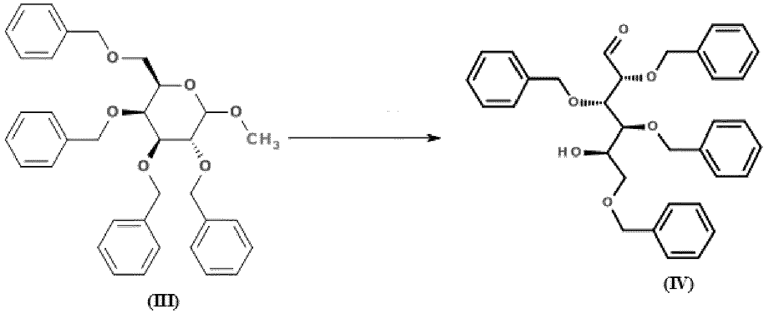 in presence of a mixture of a predefined acid at a predefined temperature;
d. purifying the compound of formula (IV) to a compound of formula (A) 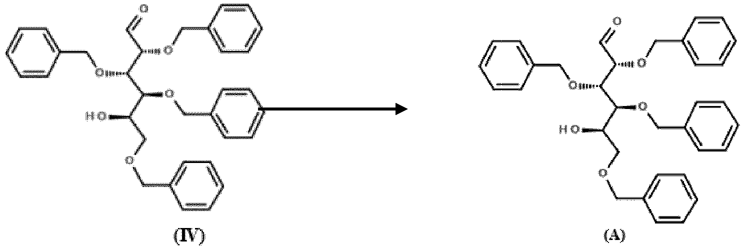 said purification process comprising the steps of:
   i. acylating the compound of formula (IV) to a compound of formula (V) 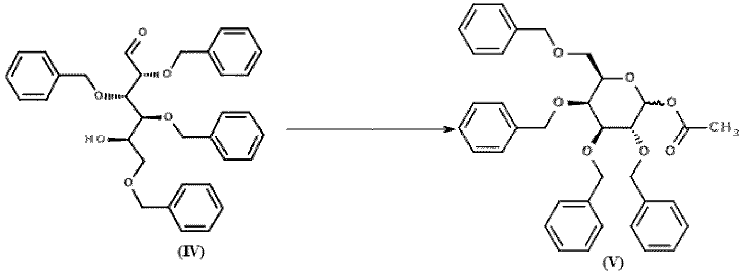 using a predefined acylating agent in presence of a predefined base and a predefined solvent at a predefined temperature; and
   ii. hydrolysis of the compound of formula (V) to compound of a formula (A) 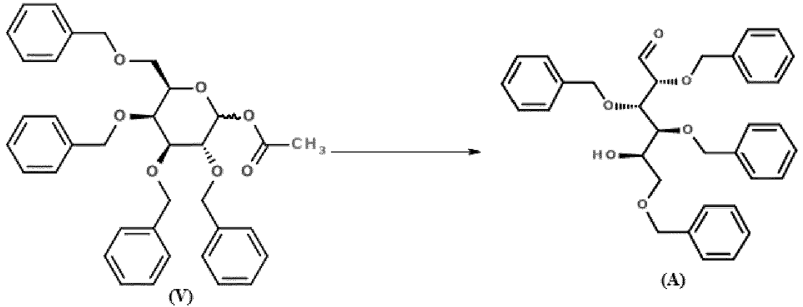 in presence of a predefined base and a predefined solvent at a predefined temperature.

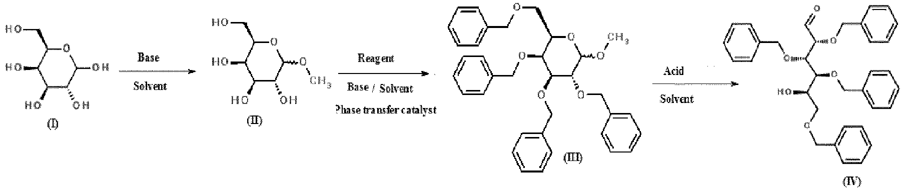

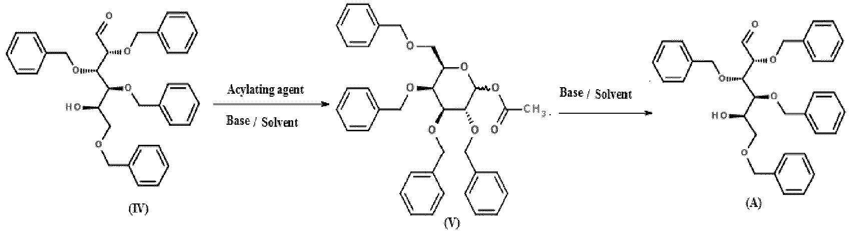

In accordance with this embodiment, a process of O-methylation of a compound of formula (I) to a compound of formula (II) in step (a.) is disclosed, wherein the process comprising O-methylation is carried out by reacting the compound of formula (I) with catalyst in predefined solvent at a predefined heating temperature.

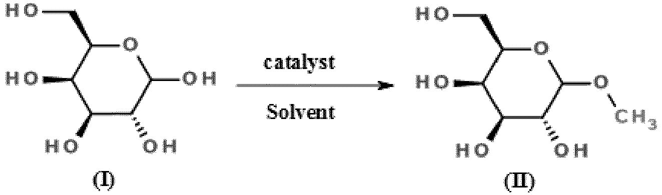

In this embodiment, the predefined catalyst is selected from the group of hydrochloric acid, and sulfuric acid. The predefined solvent selected is methanol. Further, the heating temperature ranges from 65°C to 70°C. The compound of formula (I) is β-D-Galactose. The compound of formula (II) is Methyl-D-galactopyranoside.

In accordance with this embodiment, a process of converting a compound of formula (II) to a compound of formula (III) in step (b.) is disclosed, wherein the process comprising the steps of reacting the compound of formula (II) with a predefined polar aprotic solvent in presence of a predefined phase transfer catalyst to obtain a reaction mixture; and adding a predefined base and a predefined reagent to the reaction mixture at a predefined temperature to form a compound of formula (III).

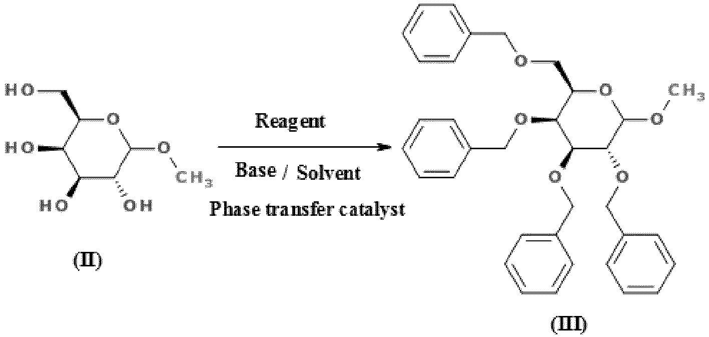

In this embodiment, the polar aprotic solvent is selected from the group of dimethyl formamide (DMF), dimethyl sulfoxide (DMSO), tetrahydrofuran, preferably (DMSO). The phase transfer catalyst is selected from the group of tetra-n-butylammonium chloride (TBAC), tetra-n-butylammonium bromide (TBAB), triethylbenzylammonium chloride (TEBA), methyltrioctylammonium chloride (Aliquat-336), cetyltriethylammonium chloride (CTEAL), benzyltributylammonium chloride (BTBAC1), tetra-n-butylammonium fluoride (TBAF), preferably tetra-n-butylammonium bromide (TBAB). The base used in the process is selected from the group of potassium hydroxide, sodium hydroxide, sodium methoxide, sodium carbonate and potassium tert-butoxide, preferably potassium hydroxide. The amount of the base used is 4.5 molar equivalent to 24 molar equivalent, preferably from 6 molar equivalent to 16 molar equivalent, and more preferably 9 molar equivalent. The reagent used is selected from the group of benzyl halide wherein the benzyl halide is selected from the group of benzyl chloride, benzyl bromide and benzyl iodide. The amount of the reagent is in the range of 5 molar equivalent to 15.5 molar equivalent, preferably 5 molar equivalent to 10 molar equivalent, and more preferably 5.7 molar equivalent. The temperature of the reaction ranges from 5°C to 30°C, preferably from 10°C to 25°C, and more preferably from 15°C to 20°C. The addition of reagent selected from benzyl halide with the base is done lot-wise being four lots over a predefined period of time from 40 minutes to 45 minutes and at a predefined temperature ranging from 15°C to 20°C in each lot. The base used in this reaction gives the product in good consistency and yield. The compound of formula (II) is methyl-D-galactopyranoside. The compound of formula (III) is 2,3,4,6-tetra-O-benzyl-methyl D-galactopyranoside.

In an embodiment, the addition of a reagent selected from benzyl halide with a predefined base is done lot-wise being in four lots over a predefined period of time from 40 minutes to 45 minutes and at a predefined temperature ranging from 15°C to 20°C in each lot.

In accordance with this embodiment, a process of converting a compound of formula (III) to a compound of formula (IV) in step (c.) is disclosed, wherein the process is carried out in presence of a predefined amount of a mixture of a predefined acid in presence of a predefined amount of water at a predefined temperature.

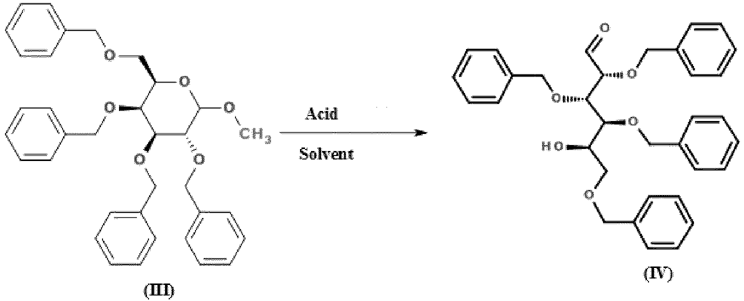

In this embodiment, the predefined acid is selected from the group of hydrobromic acid, hydrochloric acid, sulfuric acid and acetic acid and further the mixture of the predefined acid is selected from sulfuric acid and acetic acid. The predefined volume/volume percent of the mixture of the predefined acid is 0.96 : 6 to 9.6 : 17. The predefined volume/volume percent of acetic acid used ranges from 6 v/v % to 17 v/v %, preferably from 6 v/v % to 10 v/v %, and more preferably 6.75 v/v % and the predefined quantity of sulfuric acid used ranges from 0.1 to 1 molar equivalent, particularly 0.16 molar equivalent. The predefined volume/volume percent of water ranges from 1 to 9 v/v %, preferably from 1 to 3 v/v %, and more preferably 1.5 v/v %. The temperature of reaction carried out is in the range of 90°C to 110°C, preferably at a range of 100°C to 105°C. The compound of formula (III) is 2,3,4,6-tetra-O-benzyl-methyl D-galactopyranoside. The compound of formula (IV) is 2,3,4,6-tetra-O-benzyl-D-galactopyranose being crude form of compound of formula (A).

In accordance with this embodiment, a purification process of a compound of formula (IV) to a compound of formula (A) in step (d.) is disclosed, wherein the purification process comprising the step of:
i. acylating compound of formula (IV) to compound of formula (V) using a predefined acylating agent in presence of a predefined base and a predefined solvent at a predefined temperature; and
ii. hydrolysis of the compound of formula (V) to compound of formula (A) in presence of a predefined base and a predefined solvent at a predefined temperature.

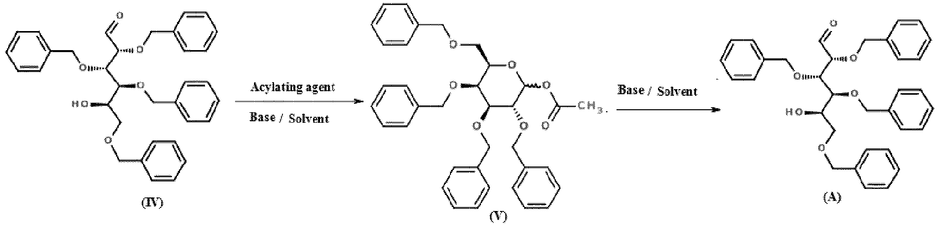

In this embodiment, in step (i.) the acylating agent is selected from the group of acetic acid, acetic anhydride. The base used is selected from the group of triethylamine and di-isopropyl ethylamine (DIPEA). The reaction is carried out in presence of a predefined solvent selected from the group of chlorinated solvents selected from the group of chloroform, dichloromethane, dichloroethane, preferably dichloromethane. The reaction temperature carried out ranges from 25°C to 30°C. In step (ii.) the base used in the reaction is selected from the group of carbonates and bicarbonates. The carbonate used is selected from the group of sodium carbonate, potassium carbonate, calcium carbonate, aluminium carbonate, ammonium carbonate, caesium carbonate, barium carbonate, magnesium carbonate, lithium carbonate. Further the bicarbonate used in the reaction is selected from the group of sodium bicarbonate, potassium bicarbonate, caesium bicarbonate, calcium bicarbonate, ammonium bicarbonate, magnesium bicarbonate. The solvent used in step (b.) is selected from the group of alcoholic solvent, alkylbenzyl solvent and/or mixtures thereof. The alcoholic solvent is selected from the group of methanol, ethanol, propanol, isopropanol, n-butanol, iso-butanol, preferably methanol; the alkylbenzyl solvent is selected from the group of toluene, o-xylene, m-xylene, p-xylene, ethylbenzene, 4-ethyl toluene, dimethyl benzene, trimethyl benzene, cymene, preferably toluene. The reaction of step (b) is carried out in presence of potassium carbonate in the mixture of alcoholic solvent and alkylbenzyl solvent selected from methanol and toluene respectively. Further the predefined volume/volume percent of methanol ranges from 4 v/v % to 8 v/v % and the predefined volume/volume percent of toluene ranges from 4 v/v % to 8 v/v % of toluene, preferably the predefined volume/volume percent of methanol is 5 v/v % and toluene is 5 v/v %. The ratio of methanol to toluene is 0.5 : 1 to 1 : 0.5.

The compound of formula (IV) is 2,3,4,6-tetra-O-benzyl-D-galactopyranose being crude form of compound of formula (A). The compound of formula (V) is 1-acetate-2,3,4,6-tetrakis-O-(phenylmethyl)-D-galactopyranose. The compound of formula (A) has HPLC purity of 97% to 99.5% with a yield of 50% to 80%.

In another embodiment, a purification process of a compound of formula (IV) to form a pure compound of formula (A) is disclosed, 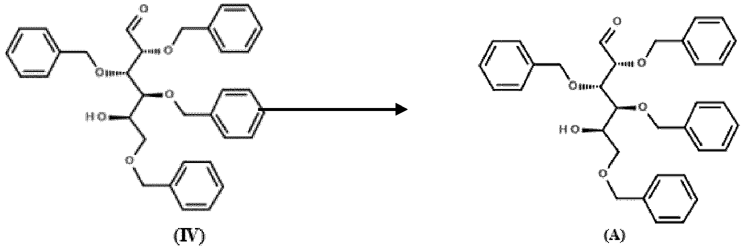 said purification process comprising:
i. acylating the compound of formula (IV) to form a compound of formula (V) using a predefined acylating agent in presence of a predefined base and a predefined solvent at a predefined temperature; and 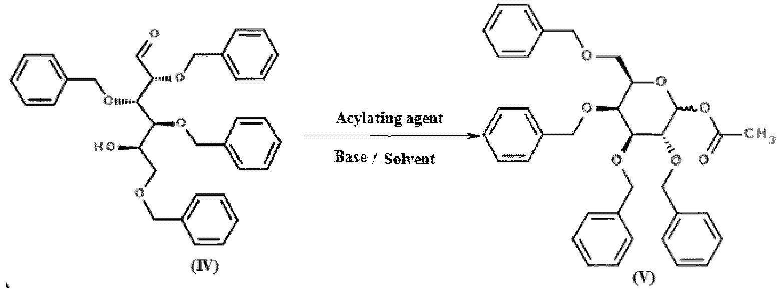
ii. hydrolysis of the compound of formula (V) to a compound of formula (A) in presence of a predefined base and a predefined solvent at a predefined temperature.

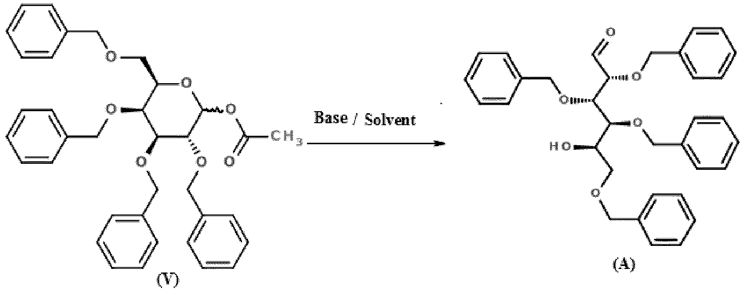

In accordance with this embodiment, in step (i.) the predefined acylating agent is selected from the group of acetic acid, acetic anhydride. The predefined base used is selected from the group of triethylamine and di-isopropylethylamine (DIPEA). The reaction is carried out in presence of a predefined solvent selected from the group of chlorinated solvents selected from the group of chloroform, dichloromethane, dichloroethane, preferably dichloromethane. The reaction temperature carried out ranges from 25°C to 30°C.

In accordance with this embodiment, in step (ii.) the predefined base used in the reaction is selected from the group of predefined carbonates and bicarbonates. The carbonate used is selected from the group of sodium carbonate, potassium carbonate, calcium carbonate, aluminium carbonate, ammonium carbonate, caesium carbonate, barium carbonate, magnesium carbonate, lithium carbonate and the bicarbonate is selected from the group of sodium bicarbonate, potassium bicarbonate, caesium bicarbonate, calcium bicarbonate, ammonium bicarbonate, magnesium bicarbonate. The solvent used in step (ii) is selected from the group of alcoholic solvent, alkylbenzyl solvent and/or mixtures thereof. The alcoholic solvent is selected from the group of methanol, ethanol, propanol, isopropanol, n-butanol and iso-butanol. The alkylbenzyl solvent is selected from the group of toluene, o-xylene, m-xylene, p-xylene, ethylbenzene, 4-ethyl toluene, dimethyl benzene, trimethyl benzene, cymene, preferably toluene.

In accordance with this embodiment, the reaction of step (ii.) is carried out in presence of potassium carbonate in a mixture of alcoholic solvent and alkylbenzyl solvent selected from methanol and toluene respectively. Further the predefined volume/volume percent of methanol ranges from 4 v/v % to 8 v/v % and toluene ranges from 4 v/v % to 8 v/v % of toluene, and preferably the predefined volume of methanol is 5 v/v % and the predefined volume/volume of toluene is 5 v/v %. The ratio of methanol to toluene is 0.5:1 to 1:0.5.

In accordance with this embodiment, the compound of formula (IV) is 2,3,4,6-tetra-O-benzyl-D-galactopyranose being crude form of compound of formula (A). The compound of formula (V) is 1-acetate-2,3,4,6-tetrakis-O-(phenylmethyl)-D-galactopyranose. The compound of formula (A) is 2,3,4,6-Tetra-O-benzyl-D-galactose. The compound of formula (A) has HPLC purity of 97% to 99.5% with yield of 50% to 80%.

In context of the present invention, the selective precipitation of acylated product occurs in preference to other impurities, due to difference in the solubility of acyl derivatives of impurities. Purity of 2,3,4,6-Tetra-O-benzyl-D-galactose is very important, as it is used as precursor for synthesis of many active pharmaceutical ingredient and intermediates. Isolation of pure 2,3,4,6-Tetra-O-benzyl-D-galactose, free of mono-, di-, tri-, penta-benzylated galactose impurities and dibenzyl ether impurity is achieved by the process of the present invention. In the present invention, mono-, di-, tri-, penta-benzylated galactose impurities and dibenzyl ether impurity are effectively removed by acylation.

The reaction scheme for a process for preparation of a compound of formula (A) is represented as follows:

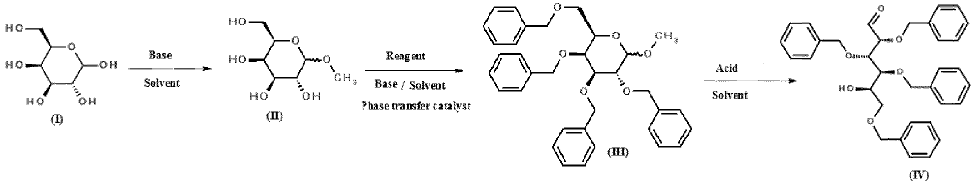

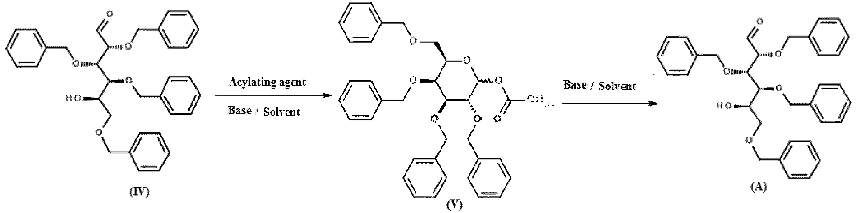

The following examples illustrate the invention, but are not limiting thereof. Variations, modifications, and enhancements to the described examples and implementations and other implementations can be made based on what is disclosed.

### EXAMPLES

Only a few examples and implementations are disclosed. Variations, modifications, and enhancements to the described examples and implementations and other implementations can be made based on what is disclosed.

Examples are set forth herein below and are illustrative of different amounts and types of reactants and reaction conditions that can be utilized in practicing the disclosure. It will be apparent, however, that the disclosure can be practiced with other amounts and types of reactants and reaction conditions than those used in the examples, and the resulting devices various different properties and uses in accordance with the disclosure above and as pointed out hereinafter.

### Example 1

### Preparation of Methyl-D-Galactopyranoside (Compound of formula (II))

Methanolic HCl was prepared by purging dry HCl gas in methanol previously cooled to 5-10°C. The purging was continued to obtain methanolic HCl of concentration 2-2.5% HCl content in 700 ml methanol. Methanolic HCl (700 ml) was added to β-D-Galactose **(I)** (100 g) with constant stirring at 25-30°C. The reaction mass was heated to 65-70°C and maintained for at least 4 hours to obtain Methyl-D-Galactopyranoside **(II)** (106 g) (Yield 98.35%)

### Example 2

### Preparation of 2,3,4,6-tetra-O-benzyl-methyl D-galactopyranoside (Compound of formula (III))

Dimethylsulfoxide (1250 ml) was charged to Methyl-D-Galactopyranoside (250 gms) as prepared example 1 at 25-30°C. The reaction mass was stirred till Methyl-D-Galactopyranoside **(II)** gets soluble in DMSO. TBAB (2.5 g) was charged to the reaction mixture. Potassium hydroxide (162.2 g) was charged to the mixture and stirred well. The mass was cooled to 15-20°C. Benzylbormide (312.5 g) was added gradually in 40-50 minutes at 15-20°C. After completion of addition reaction mass was stirred for 15 minutes at 15-20°C under inert atmosphere. Second lot of potassium hydroxide (162.2 g) was added to the reaction mass. Second lot of benzyl bromide (312.5 g) was charged in 40-45 minutes at 15-20°C. After completion of addition reaction mass was stirred for 15 minutes at 15-20°C under inert atmosphere. Third lot of potassium hydroxide (162.2 g) was added to the reaction mass. Third lot of benzyl bromide (312.5 g) was charged in 40-45 minutes at 15-20°C. After completion of addition reaction mass was stirred for 15 minutes at 15-20°C under inert atmosphere. Forth lot potassium hydroxide (162.2 g) was added to the reaction mass. Forth lot of benzyl bromide (312.5 g) was charged in 40-45 minutes at 15-20°C. After completion of addition reaction mass was stirred for 15 minutes at 15-20°C under inert atmosphere. In another round bottom flask, water (2.5 lit) was added to the mixture of acetic acid (380 g) and toluene (1250 ml) at 25-30°C. The mixture was cooled to 15-20°C and above reaction mass was added to the mixture slowly at 15-20°C. The mass was stirred for 15 minutes and aqueous layer was extracted with toluene (1250 ml) at 25-30°C. The organic layer was washed with water (1250 ml) at 25-30°C and stirred for 10 minutes. Organic layer was washed with 4% acetic acid (1250 ml) at 25-30°C and stirred for 10 minutes. Again organic layer was washed with water (1250 ml) at 25-30°C followed by washing with Brine solution (1250 ml) at 25-30°C. Toluene was distilled under vacuum at 55-60°C. The mass was degassed to yield 2,3,4,6-tetra-O-benzyl-methyl-D-galactopyranoside **(III)** (950 gm) (Yield 66.6%). HPLC Assay: 50%; Related Substances by HPLC: 45-50%

### Example 3

### Preparation of 2,3,4,6-tetra-O-benzyl-methyl D-galactopyranoside (Compound of formula (III))

Methyl-D-Galactopyranoside **(II)** (70 kg) was dissolved in dimethylsulfoxide (420 L) at 25-30°C. TBAB (0.7 kg) was charged to the solution. Lot-1 of potassium hydroxide (45.43 kg) was charged to the mixture at 25-30°C and stirred well. Chilling was applied and the mass was cooled to 15-20°C. Lot-1 of benzyl bromide (87.57 kg) was added gradually in 40-50 minutes at 15-20°C. After completion of addition reaction mass was stirred for 15 minutes at 15-20°C under inert atmosphere. Lot-II of potassium hydroxide (45.43 kg) was added to the reaction mass. Lot-II of benzyl bromide (87.57 kg) was charged in 40-45 minutes at 15-20°C. After completion of addition reaction mass was stirred for 15 minutes at 15-20°C under inert atmosphere. Lot-III of potassium hydroxide (45.43 kg) was added to the reaction mass. Lot-III of benzyl bromide (87.5 kg) was charged in 40-45 minutes at 15-20°C. After completion of addition reaction mass was stirred for 15 minutes at 15-20°C under inert atmosphere. Lot-IV potassium hydroxide (45.43 kg) was added to the reaction mass. Lot-IV of benzyl bromide (87.5 kg) was charged in 40-45 minutes at 15-20°C. After completion of addition reaction mass was stirred for 15 minutes at 15-20°C under inert atmosphere. In another round bottom flask, water (700 L) was added to the mixture of acetic acid (106.4 kg) and toluene (350 L) at 25-30°C. Chilling was applied and the mixture was cooled to 15-20°C and above reaction mass was added to the solution slowly at 15-20°C. The mass was stirred for 15 minutes and aqueous layer was extracted with toluene (350 L) at 25-30°C. The organic layer was washed with water (350 L) at 25-30°C and stirred for 10 minutes. Organic layer was separated and extracted with 10% brine solution (350 L) at 25-30°C. The organic layer was separated and Toluene was distilled out under vacuum below 55-60°C. The temperature was raised to 80-85°C to distill out traces of Toluene under vacuum to yield 2,3,4,6-tetra-O-benzyl-methyl-D-galactopyranoside **(III)** (252 kg) (Yield 80%) HPLC Assay: 63.69%; Related Substances by HPLC: 73.14%

### Example 4

### Preparation of 2,3,4,6-Tetra-O-benzyl-D-galactopyranose (Compound of formula (IV))

2,3,4,6-tetra-O-benzyl-methyl-D-galactopyranoside **(III)** (150 g) obtained in example 2 was charged to acetic acid (1.012 L) at 25-30°C. Water (225 ml) was charged at 25-30°C. Sulfuric acid 4.24g (0.16 eq) was charged at 25-30°C. The reaction mass heated to 100-105°C. The mass was maintained for 3 hours. After completion of the reaction, the mass was cooled to 25-30°C. The mass was extracted with Toluene (750 ml x 2). The toluene layer was combined and washed with water (300 ml x 2) at 25-30°C. The toluene layer was washed with 10% NaHCO₃ solution (300 ml) at 25-30°C. The toluene layer was washed with brine solution (200 ml) at 25-30°C. The layer was dried on sodium sulfate (10 g) and distilled under vacuum at 50-60°C. Degas it well to yield crude 2,3,4,6-Tetra-O-benzyl-D-galactopyranose **(IV)** (130 g) (yield 88.9% ). HPLC Purity: 52%; HPLC assay: 51.45%

### Example 5

### Preparation of 2,3,4,6-Tetra-O-benzyl-D-galactopyranose (Compound of formula (IV))

Acetic acid (796 L) was charged to 2,3,4,6-tetra-O-benzyl-methyl-D-galactopyranoside **(III)** (118 kg) at 25-30°C and stirred to get homogeneous mixture. Water (180 L) was charged at 25-30°C to the reaction mixture. Sulfuric acid 3.34kg was gradually charged to the reaction mixture at 25-30°C over period of 10-20 minutes. The reaction mass was heated to 100-105°C. The mass was maintained for 3 hours. After completion of the reaction, the mass was cooled to 25-30°C. The mass was extracted with Toluene (590 L x 2). The toluene layer was combined and washed with water (235 L x 2) at 25-30°C. The toluene layer was washed with 10% NaHCO₃ solution (235 L) at 25-30°C. The toluene layer was washed with 10% brine solution (235 L) at 25-30°C. The layer was dried on sodium sulfate (11.8 kg) and distilled under vacuum below 50-60°C. Degas it well to yield crude oil 2,3,4,6-Tetra-O-benzyl-D-galactopyranose **(IV)** (111 kg) (yield 94.06 %). HPLC Purity: 67.25%; HPLC assay: 45.08%

### Example 6

### Purification of 2,3,4,6-Tetra-O-benzyl-D-galactopyranose (Compound of formula (IV)) i) Synthesis 1-acetate-2,3,4,6-tetrakis-O-(phenylmethyl)-D-Galactopyranose (Compound of formula (V))

Crude 2,3,4,6-Tetra-O-benzyl-D-galactopyranose **(IV)** (250 gms) was charged to MDC (1500 ml) at 25-30°C under nitrogen atmosphere. Triethylamine (140 g) was charged in to the reaction mixture over a period of 20-30 minutes at 25-30°C. Acetic anhydride (128 gms) was charged to the mixture at 25-30°C over a period of 60-75 minutes and the mixture was stirred to yield homogenous mixture and maintained for 12 hours. After completion of the reaction water (500 ml) was charged in the reaction mass at 25-30°C over 30-45 minutes. MDC layer was separated and aqueous layer was again extracted with MDC (500 ml). 1% acetic acid (500 ml) was charged to MDC layer and stirred for 1 minute. MDC layer was separated and washed with water (500 ml). MDC layer was concentrated under reduced pressure and degassed for 1 hour. Oil obtained was stripped with methanol (250 ml x 2) and degassed for 30-45 minutes. Methanol (500 ml) was added to the crude oil obtained and the mixture was heated to 50-55°C for 2 hours under stirring. The mass was cooled gradually at 25-30°C and mass was stirred at 25-30°C for 4-6 hours. The solid obtained was filtered and suck dried for 30 minutes and washed with methanol (125 ml) and filtered. The solid obtained was again slurried in methanol (390 ml), heated to 50-55°C and maintained for 1 hour. The mass was allowed to cool gradually at 25 - 30°C and stirred for 2 hrs at 25 - 30°C. The wet cake was washed with methanol (65 ml) and the cake was dried at 30-40°C for 8 hours to yield 1-acetate-2,3,4,6-tetrakis-O-(phenylmethyl)-D-Galactopyranose **(V)** (118 gm) (Yield 43.8 %). The isolated 1-acetate-2,3,4,6-tetrakis-O-(phenylmethyl)-D-Galactopyranose **(V)** was further purified in methanol (1180 ml) and heated to 60-65°C and maintained for 1 hour. The mass was gradually cooled to 30-35°C. The mass was filtered after stirring for 30 minutes. The solid was washed with methanol (118 ml) and dried. The solid obtained was again purified in methanol (1180 ml) as stated above. 1-acetate-2,3,4,6-tetrakis-O-(phenylmethyl)-D-Galactopyranose **(V)** (98 gm) was obtained after drying. (HPLC purity: 99.41%)

### ii) Synthesis of 2,3,4,6-tetra-O-benzyl-D-galactose (Compound of formula (A))

To the mixture of methanol (550 ml) and Toluene (550 ml), 1-acetate-2,3,4,6-tetrakis-O-(phenylmethyl)-D-Galactopyranose **(V)** (110 g) was charged at 25-30°C. The reaction mixture was stirred for 15-30 minutes. Potassium carbonate (2.6 g) was charged to the reaction mass and stirred for 1 hour at 25-30°C. After completion of the reaction, the pH of the reaction mass was adjusted to 7-8 using ammonium chloride. The reaction mass was filtered and filtrate was distilled under reduced pressure at 45-50°C. To the residue obtained, toluene (550 ml) and water (220 ml) was charged and the mixture was stirred for 30 minutes. The layers were separated and aqueous layer was extracted again with toluene (110 ml). Toluene layers were combined and Toluene was distilled off at 45-55°C under vacuum. The residue was degassed well and 10% toluene in heptane (550 ml) was charged and the mass was stirred for 12-13 hours. The slurry was filtered and washed with 0.5 V heptane and suck dried. The solid was dried at 30-35°C under vacuum to obtain 92 grams of 2,3,4,6-tetra-O-benzyl- D-galactose **(A).** HPLC Purity: 99.8%

### Example 7

### Purification of 2,3,4,6-Tetra-O-benzyl-D-galactopyranose (Compound of formula (IV)) i) Synthesis 1-acetate-2,3,4,6-tetrakis-O-(phenylmethyl)-D-Galactopyranose (Compound of formula (V))

Crude 2,3,4,6-Tetra-O-benzyl-D-galactopyranose **(IV)** (142 kg) was charged to MDC (850 L) at 25-30°C under nitrogen atmosphere. Triethylamine (79.52 kg) was charged in to the reaction mixture over a period of 20-30 minutes at 25-30°C. Acetic anhydride (72.70 kg) was charged to the mixture at 25-30°C over a period of 60-90 minutes and the mixture was stirred to yield homogenous mixture and maintained for 8 hours. After completion of the reaction water (285 L) was charged in the reaction mass. MDC (285 L) was added to aqueous layer at 25-30°C and stirred for 10 minutes. 1% acetic acid (285 L) was charged to MDC layer and stirred for 15 minutes. MDC layer was separated and MDC was distilled out under vacuum. Methanol (140 L) was added to the reaction mass. Methanol was distilled out under vacuum below 50-55°C. The crude oil obtained was charged to methanol (140 L) and mixture was heated to 50-55°C for 2 hours under stirring. Methanol was distilled under vacuum under below 50-55°C. Methanol (570 L) was charged in the reaction mass, heated to 50-55°C and maintained for 2 hours. The mass was gradually cooled to 25-30°C and maintained for 4-6 hours. The mass was centrifuged and washed with methanol (70 L), spin dried to yield 60 kg of the title compound. The wet cake obtained was dried at 35-40°C. Dry weight was 48 kg (HPLC purity >90%). The isolated 1-acetate-2,3,4,6-tetrakis-O-(phenylmethyl)-D-Galactopyranose **(V)** was further purified in methanol (480 L) and heated to 60-65°C and maintained for 1 hour. The mass was gradually cooled to 30-35°C. The mass was filtered after stirring for 30 minutes. The solid was washed with methanol (48 L) and dried. The solid obtained was again purified in methanol (480 L) as stated above, 1-acetate-2,3,4,6-tetrakis-O-(phenylmethyl)-D-Galactopyranose **(V)** (42 kg) was obtained after drying. (HPLC purity: 98.8%)

### ii) Synthesis of 2,3,4,6-tetra-O-benzyl-D-galactose (Compound of formula (A))

To the mixture of methanol (525 L) and Toluene (625 L ml), 1-acetate-2,3,4,6-tetrakis-O-(phenylmethyl)-D-Galactopyranose **(V)** (125 kg) was charged at 25-30°C under nitrogen. The reaction mixture was stirred for 15-30 minutes. Potassium carbonate (2.87 kg) was charged to the reaction mass and stirred for 1 hour at 25-30°C. After completion of the reaction, the pH of the reaction mass was adjusted to 7-8 using ammonium chloride. The reaction mass was filtered and filtrate was distilled under vacuum below 50°C. To the residue obtained, toluene (625 L) and water (250 L) was charged and the mixture was stirred for 30 minutes. The layers were separated and aqueous layer was extracted again with toluene (125 L). Toluene layers were combined and Toluene was distilled off below 50°C under vacuum. The residue was degassed well and heptane (625 L) and Toluene (10 L) was charged and the mass was stirred for 12-13 hours. The slurry was filtered and washed with heptane (63 L) and suck dried. The wet cake obtained was added to mixture of Toluene (35 L) and Heptane (690 L) at 25-30°C. The mass was stirred for 1- 2 hours at 25-30°C under nitrogen. The mass was centrifuged and spin dried. The wet cake was washed with heptane (69 L). The product was dried at 30-35°C to yield 2,3,4,6-tetra-O-benzyl- D-galactose (94 kg) **(A).** HPLC Purity: 99.9%

In the context of the present invention, the process of the present invention is an eco-friendly and a cost effective process. Further mono-, di-, tri-, penta-benzylated galactose impurities and dibenzyl ether impurity are effectively removed by acylation by the process of the present invention. The process of the present invention is an easy process to achieve purity of 2,3,4,6-tetra-O-benzyl-D-galactose with general purification techniques. Further the separation of impurities from the product is very easy and the process of the present invention results in high yield of the end product with maximum purity.

The foregoing description of specific embodiments of the present invention has been presented for purposes of illustration and description. They are not intended to be exhaustive or to limit the present invention to the precise forms disclosed, and obviously many modifications and variations are possible in light of the above teaching.

The embodiments were chosen and described in order to best explain the principles of the present invention and its practical application, to thereby enable others, skilled in the art to best utilize the present invention and various embodiments with various modifications as are suited to the particular use contemplated.

It is understood that various omission and substitutions of equivalents are contemplated as circumstance may suggest or render expedient, but such are intended to cover the application or implementation as far as they are within the scope of the present invention, which is defined by the claims.

## Claims

1. A process for preparation of a compound of formula (A) comprising 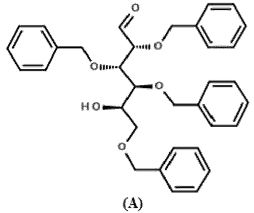
a. O-methylation of a compound of formula (I) to a compound of formula (II) 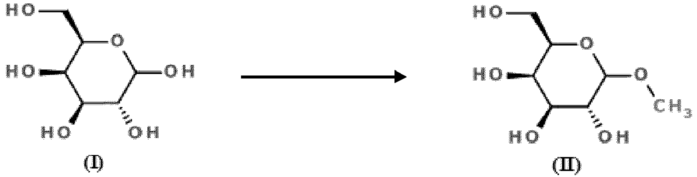 in presence of a predefined catalyst and a predefined solvent at a predefined heating temperature;
b. reacting the compound of formula (II) in a predefined polar aprotic solvent in presence of a predefined phase transfer catalyst to obtain a reaction mixture; and adding a predefined base and a predefined reagent to the reaction mixture at a predefined temperature to form a compound of formula (III); 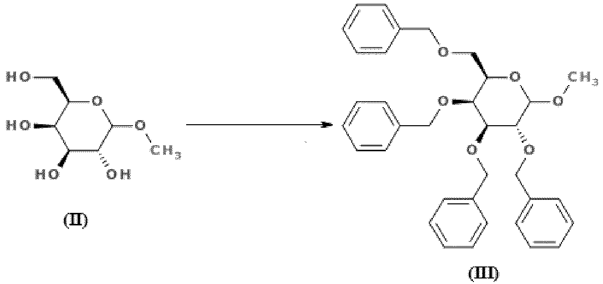
c. converting the compound of formula (III) to a compound of formula (IV) 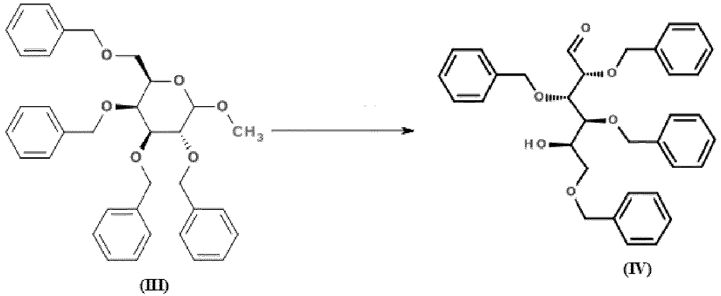 in presence of a mixture of a predefined acid at a predefined temperature;
d. purifying the compound of formula (IV) to a compound of formula (A) 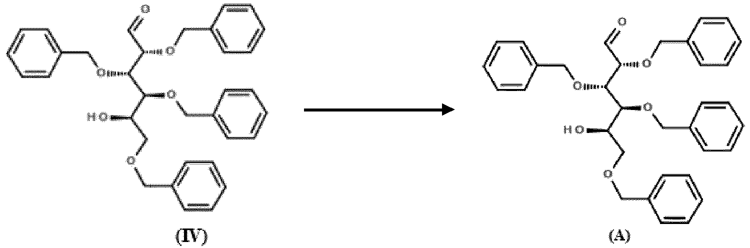 said purification process comprising the steps of:
i. acylating the compound of formula (IV) to form a compound of formula (V) 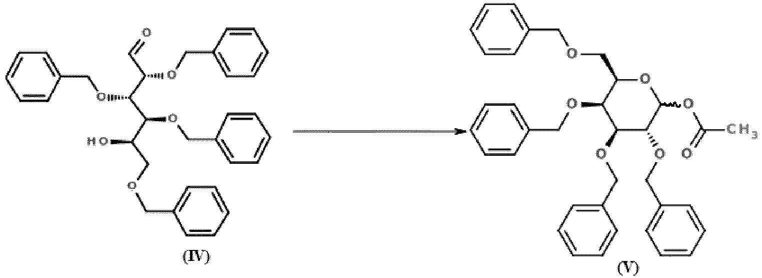 using a predefined acylating agent in presence of a predefined base and a predefined solvent at a predefined temperature; and
ii. hydrolysis of the compound of formula (V) to form the compound of formula (A) 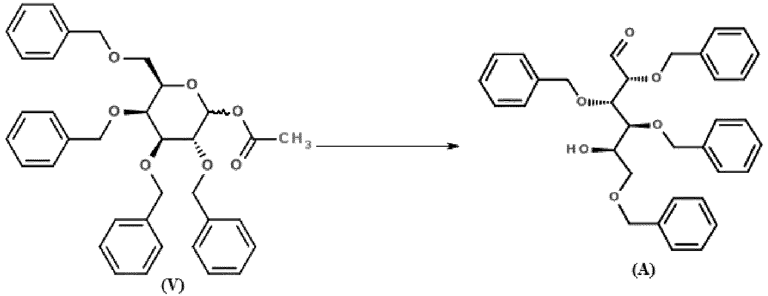 in presence of a predefined base and a predefined solvent at a predefined temperature.

2. The process as claimed in claim 1, wherein the predefined catalyst in step (a.) is selected from the group of hydrochloric acid and sulfuric acid.

3. The process as claimed in claim 1, wherein the predefined polar aprotic solvent in step (b.) is selected from the group of dimethyl formamide (DMF), dimethyl sulfoxide (DMSO), tetrahydrofuran, preferably dimethyl sulfoxide (DMSO).

4. The process as claimed in claim 1, wherein the predefined phase transfer catalyst in step (b.) is selected from the group of tetra-n-butylammonium chloride (TBAC), tetra-n-butylammonium bromide (TBAB), triethylbenzylammonium chloride (TEBA), methyltrioctylammonium chloride (Aliquat-336), cetyltriethylammonium chloride (CTEAL), benzyltributylammonium chloride (BTBAC1), tetra-n-butylammonium fluoride (TBAF), preferably tetra-n-butylammonium bromide (TBAB).

5. The process as claimed in claim 1, wherein the predefined base in step (b.) is selected from the group of potassium hydroxide, sodium hydroxide, sodium methoxide, sodium carbonate and potassium tert-butoxide, preferably potassium hydroxide and/or wherein the amount of base in step (b.) is in the range of 4.5 molar equivalent to 24 molar equivalent.

6. The process as claimed in claim 1, wherein the predefined reagent in step (b.) is selected from the group of benzyl halide wherein the benzyl halide is selected from the group of benzyl chloride, benzyl bromide and benzyl iodide.

7. The process as claimed in claim 1, wherein the addition of the predefined reagent with the predefined base in step (b.) is done in lot over a predefined period of time from 40 minutes to 45 minutes and at a predefined temperature ranging from 15°C to 20°C in each lot.

8. The process as claimed in claim 1, wherein the predefined acid in step (c.) is selected from the group of hydrobromic acid, hydrochloric acid, sulfuric acid and acetic acid; optionally wherein the predefined mixture of the predefined acid is selected from sulfuric acid and acetic acid.

9. The process as claimed in claim 1, wherein the predefined acylating agent in step (d.) (i.) is selected from the group of acetic acid and acetic anhydride.

10. The process as claimed in claim 1, wherein the predefined base in step (d.) (i.) is selected from the group of triethylamine and di-isopropylethylamine (DIPEA).

11. The process as claimed in claim 1, wherein the predefined solvent in step (d.) (i.) is selected from the group of chlorinated solvents selected from the group of chloroform, dichloromethane, dichloroethane, preferably dichloromethane.

12. The process as claimed in claim 1, wherein the predefined temperature in step (d.) (i.) is in the range from 25°C to 30°C.

13. The process as claimed in claim 1, wherein the predefined base in step (d.) (ii.) is selected from the group of predefined carbonates selected from the group of sodium carbonate, potassium carbonate, calcium carbonate, aluminium carbonate, ammonium carbonate, caesium carbonate, barium carbonate, magnesium carbonate, lithium carbonate and bicarbonates selected from the group of sodium bicarbonate, potassium bicarbonate, caesium bicarbonate, calcium bicarbonate, ammonium bicarbonate, magnesium bicarbonate.

14. The process as claimed in claim 1, wherein the predefined solvent in step (d.) (ii.) is selected from the group of solvent methanol, ethanol, propanol, isopropanol, n-butanol, iso-butanol, toluene, o-xylene, m-xylene, p-xylene, ethylbenzene, 4-ethyl toluene, dimethyl benzene, trimethyl benzene and cymene and/or mixtures thereof; or wherein in step (d.) (ii) a predefined mixture of methanol and toluene and wherein the predefined volume/volume percent of methanol ranges from 4 v/v % to 8 v/v % and toluene ranges from 4 v/v % to 8 v/v %; optionally wherein the methanol to toluene ratio is 0.5:1 to 1:0.5.

## Patentansprüche

1. Verfahren zur Herstellung einer Verbindung der Formel (A), umfassend: 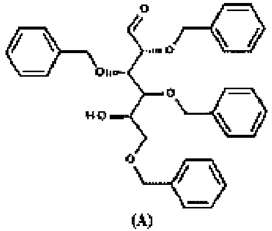
a. O-Methylierung einer Verbindung der Formel (I) an eine Verbindung der Formel (II) 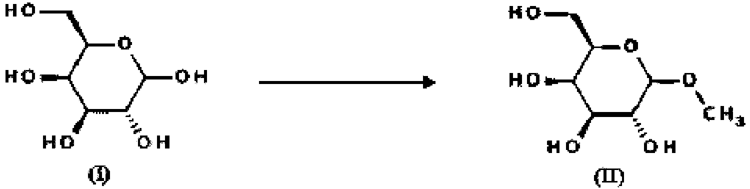 in Gegenwart eines vordefinierten Katalysators und eines vordefinierten Lösungsmittels bei einer vordefinierten Erwärmungstemperatur;
b. Umsetzen der Verbindung der Formel (II) in einem vordefinierten polar-aprotischen Lösungsmittel in Gegenwart eines vordefinierten Phasentransferkatalysators, um eine Reaktionsmischung zu erhalten; und Zufügen einer vordefinierten Base und eines vordefinierten Reagenzes zu der Reaktionsmischung bei einer vordefinierten Temperatur, um eine Verbindung der Formel (III) zu bilden; 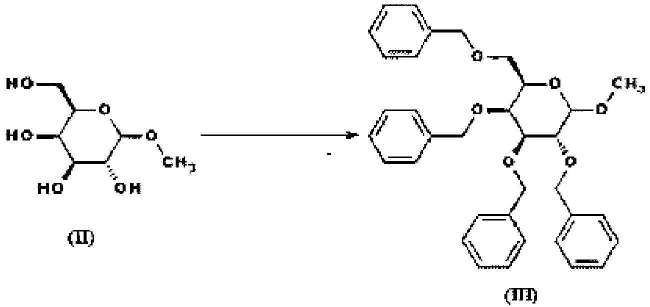
c. Umwandeln einer Verbindung der Formel (III) in eine Verbindung der Formel (IV) 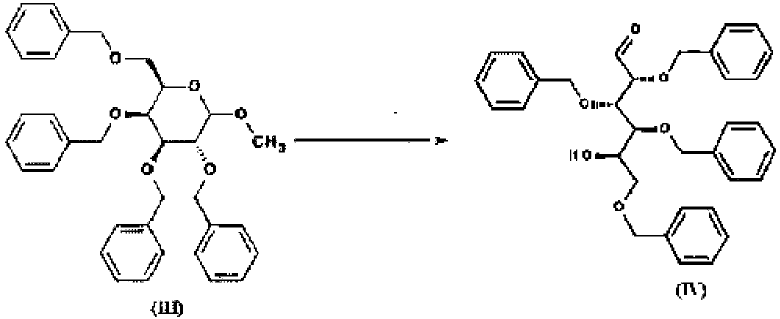 in Gegenwart einer Mischung einer vordefinierten Säure bei einer vordefinierten Temperatur;
d. Reinigen der Verbindung der Formel (IV) zu einer Verbindung der Formel (A), 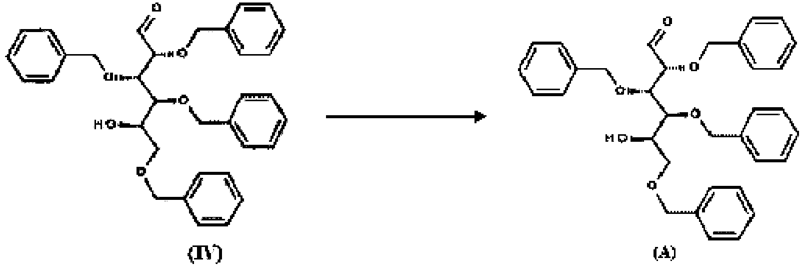 wobei das Reinigungsverfahren die Schritte umfasst:
i. Acylieren der Verbindung der Formel (IV), um eine Verbindung der Formel (V) zu bil - den, 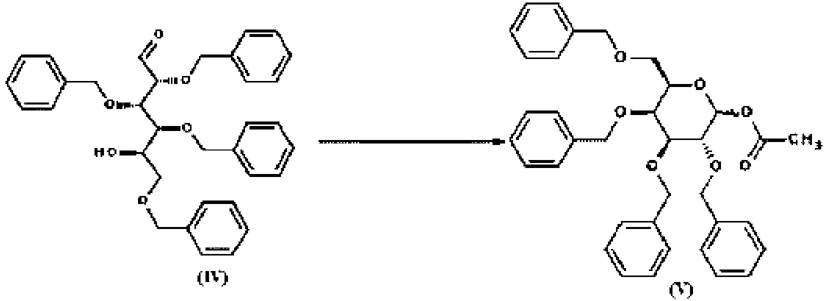 unter Verwendung eines vordefinierten Acylierungsmittels in Gegenwart einer vordefinierten Base und eines vordefinierten Lösungsmittels bei einer vordefinierten Temperatur; und
ii. Hydrolysieren der Verbindung der Formel (V), um die Verbindung der Formel (A) zu bilden, 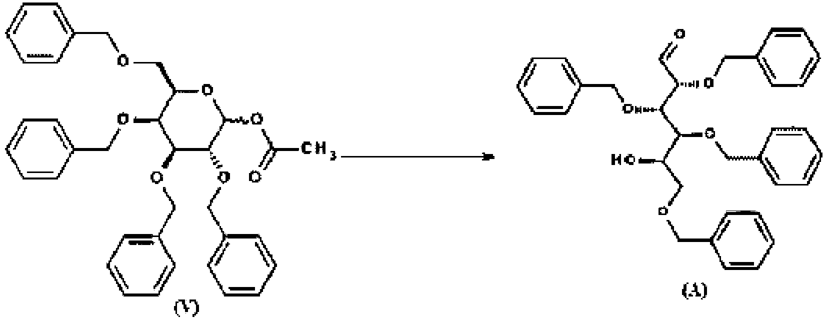 in Gegenwart einer vordefinierten Base und eines vordefinierten Lösungsmittels bei einer vordefinierten Temperatur.

2. Verfahren nach Anspruch 1, wobei der vordefinierte Katalysator in Schritt (a.) ausgewählt ist aus der Gruppe von Salzsäure und Schwefelsäure.

3. Verfahren nach Anspruch 1, wobei das vordefinierte polar-aprotische Lösungsmittel in Schritt (b.) ausgewählt ist aus der Gruppe von Dimethylformamid (DMF), Dimethylsulfoxid (DMSO), Tetrahydrofuran, vorzugsweise Dimethylsulfoxid (DMSO).

4. Verfahren nach Anspruch 1, wobei der vordefinierte Phasentransferkatalysator in Schritt (b.) ausgewählt ist aus der Gruppe von Tetra-n-butylammoniumchlorid (TBAC), Tetra-n-butylammoniumbromid (TBAB), Triethylbenzylammoniumchlorid (TEBA), Methyltrioctylammoniumchlorid (Aliquat-336), Cetyltriethylammoniumchlorid (CTEAL), Benzyltributylammoniumchlorid (BTBACl), Tetra-n-butylammoniumfluorid (TBAF), vorzugsweise Tetra-n-butylammoniumbromid (TBAB).

5. Verfahren nach Anspruch 1, wobei die vordefinierte Base in Schritt (b.) ausgewählt ist aus der Gruppe von Kaliumhydroxid, Natriumhydroxid, Natriummethoxid, Natriumcarbonat und Kalium-tert-butoxid, vorzugsweise Kaliumhydroxid und/oder wobei die Menge der Base in Schritt (b.) im Bereich von 4,5 Moläquivalent bis 24 Moläquivalent liegt.

6. Verfahren nach Anspruch 1, wobei das vordefinierte Reagenz in Schritt (b.) ausgewählt ist aus der Gruppe von Benzylhalogenid, wobei das Benzylhalogenid ausgewählt ist aus der Gruppe von Benzylchlorid, Benzylbromid und Benzyliodid.

7. Verfahren nach Anspruch 1, wobei die Zugabe des vordefinierten Reagenzes mit der vordefinierten Base in Schritt (b.) in einer Charge über einen vordefinierten Zeitraum von 40 Minuten bis 45 Minuten und in einem vordefinierten Temperaturbereich von 15 °C bis 20 °C in jeder Charge durchgeführt wird.

8. Verfahren nach Anspruch 1, wobei die vordefinierte Säure in Schritt (c.) ausgewählt ist aus der Gruppe von Bromwasserstoffsäure, Salzsäure, Schwefelsäure und Essigsäure; wobei die vordefinierte Mischung der vordefinierten Säure gegebenenfalls ausgewählt ist aus Schwefelsäure und Essigsäure.

9. Verfahren nach Anspruch 1, wobei das vordefinierte Acylierungsmittel in Schritt (d.)(i.) ausgewählt ist aus der Gruppe von Essigsäure und Essigsäureanhydrid.

10. Verfahren nach Anspruch 1, wobei die vordefinierte Base in Schritt (d.)(i.) ausgewählt ist aus der Gruppe von Triethylamin und Diisopropylethylamin (DIPEA).

11. Verfahren nach Anspruch 1, wobei das vordefinierte Lösungsmittel in Schritt (d.)(i.) ausgewählt ist aus der Gruppe von chlorierten Lösungsmitteln ausgewählt aus der Gruppe von Chloroform, Dichlormethan, Dichlorethan, vorzugsweise Dichlormethan.

12. Verfahren nach Anspruch 1, wobei die vordefinierte Temperatur in Schritt (d.)(i.) im Bereich von 25 °C bis 30 °C liegt.

13. Verfahren nach Anspruch 1, wobei die vordefinierte Base in Schritt (d.)(ii.) ausgewählt ist aus der Gruppe von vordefinierten Carbonaten ausgewählt aus der Gruppe von Natriumcarbonat, Kaliumcarbonat, Calciumcarbonat, Aluminiumcarbonat, Ammoniumcarbonat, Cäsiumcarbonat, Bariumcarbonat, Magnesiumcarbonat, Lithiumcarbonat und Bicarbonaten ausgewählt aus der Gruppe von Natriumbicarbonat, Kaliumbicarbonat, Cäsiumbicarbonat, Calciumbicarbonat, Ammoniumbicarbonat, Magnesiumbicarbonat.

14. Verfahren nach Anspruch 1, wobei das vordefinierte Lösungsmittel in Schritt (d.)(ii.) ausgewählt ist aus der Gruppe von Lösungsmittel Methanol, Ethanol, Propanol, Isopropanol, n-Butanol, Isobutanol, Toluol, o-Xylol, m-Xylol, p-Xylol, Ethylbenzol, 4-Ethyltoluol, Dimethylbenzol, Trimethylbenzol und Cymol und/oder Mischungen davon; oder wobei in Schritt (d.)(ii) eine vordefinierte Mischung aus Methanol und Toluol, und wobei die vordefinierten Prozent (Volumen/Volumen) an Methanol im Bereich von 4 Vol./Vol. % bis 8 Vol./Vol. % liegen und Toluol im Bereich von 4 Vol./Vol. % bis 8 Vol./Vol. % liegt; wobei gegebenenfalls das Verhältnis von Methanol zu Toluol 0,5:1 bis 1:0,5 beträgt.

## Revendications

1. Procédé de préparation d'un composé de la formule (A) comprenant 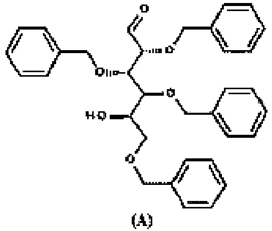
a. la O-méthylation d'un composé de la formule (I) en un composé de la formule (II) 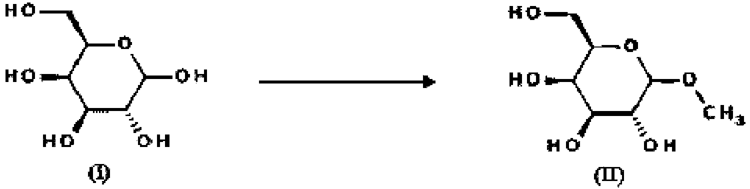 en présence d'un catalyseur prédéfini et d'un solvant prédéfini à une température de chauffage prédéfinie ;
b. la réaction du composé de la formule (II) dans un solvant aprotique polaire prédéfini en présence d'un catalyseur de transfert de phase prédéfini pour obtenir un mélange réactionnel ; et l'ajout d'une base prédéfinie et d'un réactif prédéfini au mélange réactionnel à une température prédéfinie pour former un composé de la formule (III) ; 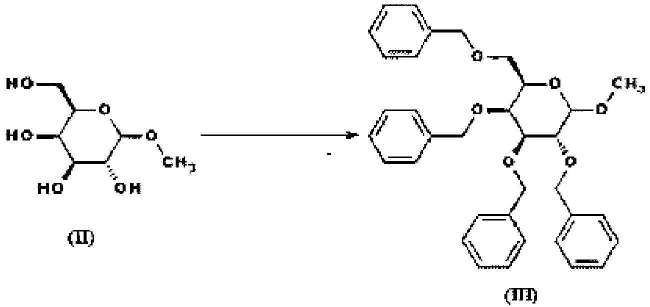
c. la conversion du composé de la formule (III) en un composé de la formule (IV) 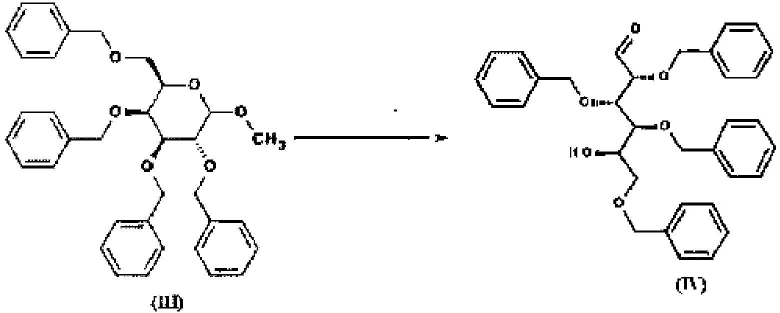 en présence d'un mélange d'un acide prédéfini à une température prédéfinie ;
d. la purification du composé de la formule (IV) en un composé de la formule (A) 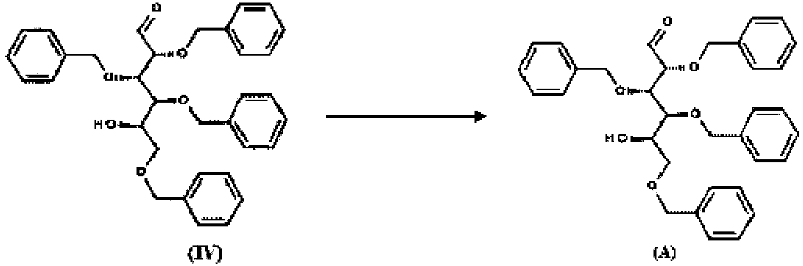 ledit procédé de purification comportant les étapes comprenant :
i. l'acylation du composé de la formule (IV) pour former un composé de la formule (V) 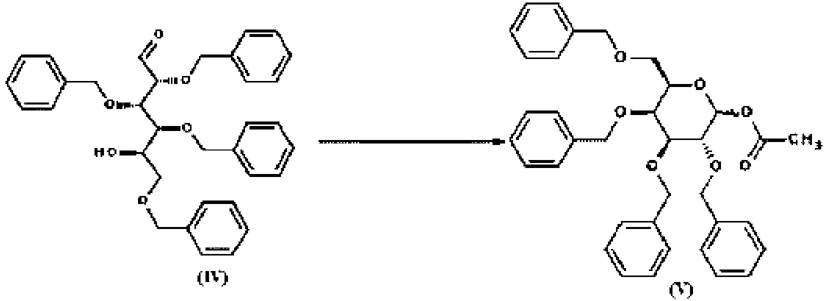 l'utilisation d'un agent d'acylation prédéfini en présence d'une base prédéfinie et d'un solvant prédéfini à une température prédéfinie ; et
ii. l'hydrolyse du composé de la formule (V) pour former le composé de la formule (A) 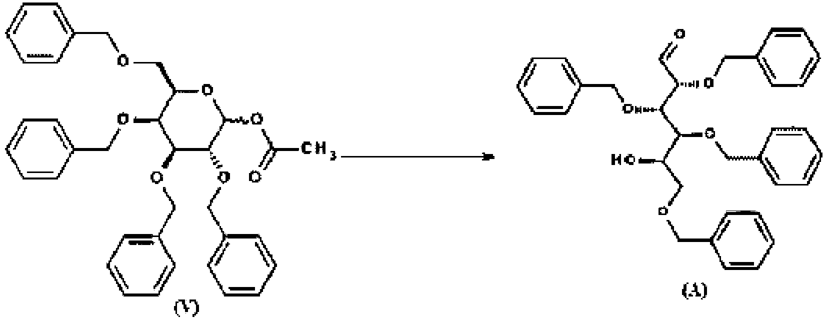 en présence d'une base prédéfinie et d'un solvant prédéfini à une température prédéfinie.

2. Procédé selon la revendication 1, dans lequel le catalyseur prédéfini de l'étape (a.) est choisi parmi le groupe constitué de l'acide chlorhydrique et de l'acide sulfurique.

3. Procédé selon la revendication 1, dans lequel le solvant aprotique polaire prédéfini de l'étape (b.) est choisi parmi le groupe constitué du diméthylformamide (DMF), du diméthylsulfoxyde (DMSO), du tétrahydrofuranne, de préférence du diméthylsulfoxyde (DMSO).

4. Procédé selon la revendication 1, dans lequel le catalyseur de transfert de phase prédéfini de l'étape (b.) est choisi parmi le groupe constitué du chlorure de tétra-n-butylammonium (TBAC), du bromure de tétra-n-butylammonium (TBAB), du chlorure de triéthylbenzylammonium (TEBA), du chlorure de méthyltrioctylammonium (Aliquat-336), du chlorure de cétyltriéthylammonium (CTEAL), du chlorure de benzyltributylammonium (BTBAC1), du fluorure de tétra-n-butylammonium (TBAF), de préférence du bromure de tétra-n-butylammonium (TBAB).

5. Procédé selon la revendication 1, dans lequel la base prédéfinie de l'étape (b.) est choisie dans le groupe constitué de l'hydroxyde de potassium, de l'hydroxyde de sodium, du méthylate de sodium, du carbonate de sodium et du tert-butylate de potassium, de préférence de l'hydroxyde de potassium et/ou dans lequel la quantité de base de l'étape (b.) se situe dans la plage de 4,5 équivalents molaires à 24 équivalents molaires.

6. Procédé selon la revendication 1, dans lequel le réactif prédéfini de l'étape (b.) est choisi dans le groupe constitué d'un halogénure de benzyle, dans lequel l'halogénure de benzyle est choisi dans le groupe constitué du chlorure de benzyle, du bromure de benzyle et de l'iodure de benzyle.

7. Procédé selon la revendication 1, dans lequel l'addition du réactif prédéfini avec la base prédéfinie de l'étape (b.) est effectuée en lot sur une période prédéfinie allant de 40 minutes à 45 minutes et à une température prédéfinie allant de 15 °C à 20 °C dans chaque lot.

8. Procédé selon la revendication 1, dans lequel l'acide prédéfini de l'étape (c.) est choisi dans le groupe constitué de l'acide bromhydrique, de l'acide chlorhydrique, de l'acide sulfurique et de l'acide acétique ; éventuellement dans lequel le mélange prédéfini de l'acide prédéfini est choisi parmi l'acide sulfurique et l'acide acétique.

9. Procédé selon la revendication 1, dans lequel l'agent d'acylation prédéfini de l'étape (d.) (i.) est choisi dans le groupe constitué de l'acide acétique et de l'anhydride acétique.

10. Procédé selon la revendication 1, dans lequel la base prédéfinie de l'étape (d.) (i.) est choisie dans le groupe constitué de la triéthylamine et de la di-isopropyléthylamine (DIPEA).

11. Procédé selon la revendication 1, dans lequel le solvant prédéfini de l'étape (d.) (i.) est choisi dans le groupe constitué de solvants chlorés choisis dans le groupe constitué du chloroforme, du dichlorométhane, du dichloroéthane, de préférence du dichlorométhane.

12. Procédé selon la revendication 1, dans lequel la température prédéfinie de l'étape (d.) (i.) se situe dans la plage allant de 25 °C à 30 °C.

13. Procédé selon la revendication 1, dans lequel la base prédéfinie de l'étape (d.) (ii.) est choisie dans le groupe constitué de carbonates prédéfinis choisis dans le groupe constitué du carbonate de sodium, du carbonate de potassium, du carbonate de calcium, du carbonate d'aluminium, du carbonate d'ammonium, du carbonate de césium, du carbonate de baryum, du carbonate de magnésium, du carbonate de lithium et de bicarbonates choisis dans le groupe constitué du bicarbonate de sodium, du bicarbonate de potassium, du bicarbonate de césium, du bicarbonate de calcium, du bicarbonate d'ammonium, du bicarbonate de magnésium.

14. Procédé selon la revendication 1, dans lequel le solvant prédéfini de l'étape (d.) (ii.) est choisi dans le groupe constitué des solvants suivants : méthanol, éthanol, propanol, isopropanol, n-butanol, isobutanol, toluène, o-xylène, m-xylène, p-xylène, éthylbenzène, 4-éthyltoluène, diméthylbenzène, triméthylbenzène et cymène et/ou de mélanges de ceux-ci ; ou dans lequel dans l'étape (d.) (ii), un mélange prédéfini de méthanol et de toluène et dans lequel le pourcentage volume/volume prédéfini de méthanol se situe entre 4 % v/v et 8 % v/v et celui du toluène se situe entre 4 % v/v et 8 % v/v ; éventuellement dans lequel le rapport méthanol au toluène varie de 0.5:1 à 1:0.5.
